# EUROPEAN PATENT APPLICATION

(11) **EP 4 413 993 A1**
(43) Date of publication of application: **14.08.2024**
(21) Application number: 23156176.2
(22) Date of filing: 10.02.2023
(51) Int. Cl.: A61K 38/18, C07K 14/47, C07K 14/475, C07K 1/113, C12P 21/06, G01N 30/34

(54) **METHOD OF OBTAINING RECOMBINANT HUMAN BRAIN-DERIVED NEUROTROPHIC FACTOR**

(71) Applicant: Dompe' Farmaceutici S.P.A., 20122 Milan (IT)
(72) Inventor: MARTIN, Franck, 60100 L'Aquila (IT); CATTANI, Franca, 67100 L'Aquila (IT); SPACCAPANICCIA, Elisa, 67100 L'Aquila (IT); CAZZORLA, Tiziano, 67100 L'Aquila (IT); MAFFEI, Mariano, 67100 L'Aquila (IT); ROSSETTI, Daniela, 67100 L'Aquila (IT); ANTONANGELI, Maria Irene, 67100 L'Aquila (IT)
(74) Representative: Dompé farmaceutici Spa

(57) **Abstract**

The present invention relates to a human proBDNF mutein and to a method of recombinantly producing a biologically active human brain-derived neurotrophic factor (BDNF).

## Description

### FIELD OF THE INVENTION

The present invention relates to a human proBDNF mutein and to a method of recombinantly producing a human brain-derived neurotrophic factor.

### STATE OF THE ART

Neurotrophins are a family of growth factors crucial for the regulation of neuronal maintenance, differentiation, and survival. In particular, neurotrophin signaling regulates a diverse set of neural processes including differentiation, neurite outgrowth, axon pruning, apoptosis, and cell survival during development, adulthood, and following injury to the nervous system.

Neurotrophins mediate these actions by binding different receptors: tropomyosin-related kinase (Trk) receptor tyrosine kinases, the p75 neurotrophin receptor, and sortilin family members. The human neurothrophin family is composed of nerve growth factor (NGF), brain-derived neurotrophic factor (BDNF), neurotrophin-3 (NT-3) and neurotrophin-4 (NT-4). Each of these structurally related neurotrophins is synthesized as a precursor prior to proteolytic cleavage to produce a mature neurotrophin (Huang E.J. et al., Annu. Rev. Biochem. 2003; 72: 609-642).

Mature human BDNF has 119 amino acid residues (SEQ ID NO:1, Gray K. et al., FEBS letters. 2008; 582 (6): 907-10), which represent the functional domain of the protein, where three disulphide bonds are located (Robinson R.C. et al., Protein Science. 1999;8(12):2589-97). The protein gene of BDNF is on 11 p human chromosome (Tian F. et al, Amino acids. 2010;38(4):1067-74).

BDNF binds at least two cell surface receptors that are capable of responding to this growth factor, TrkB and p75. This signaling molecule has been well-documented for its ability to regulate neuronal plasticity, cell growth, proliferation, cell survival, and long-term memory. In particular, it has been shown to act on certain neurons of the central nervous system and the peripheral nervous system, helping to support survival of existing neurons, and encouraging growth and differentiation of new neurons and synapses. In the brain it is active in the hippocampus, cortex, and basal forebrain-areas vital to learning, memory, and higher thinking. BDNF is also expressed in the retina, kidneys, prostate, motor neurons, and skeletal muscle, and is also found in saliva.

Human BDNF can be considered a promising therapeutic molecule.

To be developed clinically, human BDNF must meet current regulations regarding the production of clinically acceptable therapeutic products. In particular, it is necessary to produce human BDNF that is characterized by a sufficiently high degree of purity and a consistent composition and activity profile.

An optimal method of production of human BDNF for therapeutic use must therefore ensure the production of a molecule satisfying these requirements and be suitable to be adapted to large-scale implementation.

Some methods of obtaining and/or producing BDNF have been developed, but they are characterized by several drawbacks, with complex operational flow and do not result in a protein with suitable features for therapeutic use.

Thus, it is felt the need of developing an improved, simple method of producing biologically active human BDNF, able to ensure a high degree of purity of the final product and satisfactory and reproducible biological activity.

### SUMMARY OF THE INVENTION

The present invention relates to a human proBDNF mutein having the amino acid sequence of SEQ ID NO:3, wherein X1, X2 and X3 are selected from non-basic amino acids and histidine and X4 is selected from Arginine and Lysine.

Another object of the invention is a nucleic acid encoding the human proBDNF mutein as defined above.

A further object of the present invention is a method of producing a human BDNF comprising the steps of:
(i) providing a solution of the human proBDNF mutein as defined above;
(ii) purifying the human proBDNF mutein from the solution provided in step (i), thereby obtaining a solution of purified human proBDNF mutein; and
(iii) cleaving the human proBDNF mutein in said solution to obtain human BDNF.

A further object of the present invention is a human BDNF obtained, or obtainable, by the method described above.

### BRIEF DESCRIPTION OF THE FIGURES

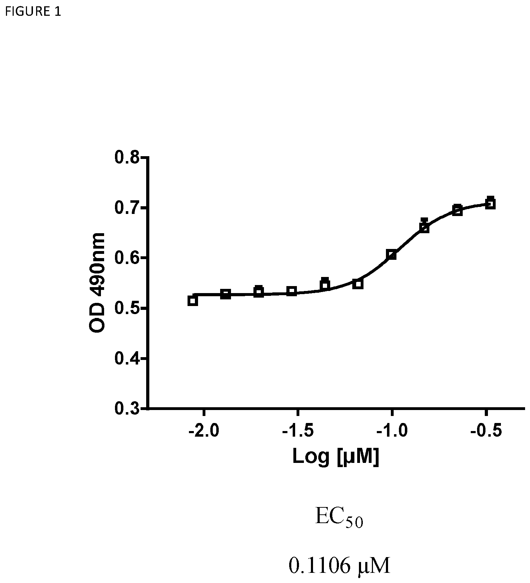

Figure 1 shows concentration-response (0.23 to 9 µg/mL) proliferation curve of C6 cells incubated with BDNF for 48h.

### DEFINITIONS

The term "human wild type BDNF" or "human BDNF" refer to the human form of the protein BDNF, having the amino acid sequence of SEQ ID NO: 1

The term "human wild type proBDNF" or "human proBDNF" refers to the precursor of human BDNF, having the amino acid sequence of SEQ ID NO: 2.

The term "human proBDNF mutein" or "human proBDNF mutant" refers to a human proBDNF containing one or more amino acids replacements.

The term "correctly folded" as used herein means:
- when referred to human wild type BDNF/human BDNF, a human wild type BDNF with a three-dimensional structure corresponding to that of the native biologically active human BDNF.
- when referred to human proBDNF mutein, human proBDNF mutein with a three-dimensional structure that results in the formation of a correctly folded human BDNF upon cleavage of the pro-sequence with a serine protease.

The term "non-basic amino acid" refers to any amino acid other than a basic amino acid.

### DETAILED DESCRIPTION OF THE INVENTION

As will be explained more in details in the experimental section, the inventors have developed a method of recombinantly producing human brain-derived neurotrophic factor (BDNF) which is advantageous over the methods disclosed in the prior art, since it is simple and allows to obtain biologically active BDNF with high purity and high yields. This is achieved by expressing a human proBDNF mutein as described below as a precursor of BDNF and by using specific process parameters and materials as will be discussed below.

Accordingly, a first object of the invention is a human proBDNF mutein having the amino acid sequence of SEQ ID NO: 3:
MAPMKEANIRGQGGLAYPGVRTHGTLESVNGPKAGSRGLTSLADTFEHVIEELLDEDQ KVRPNEENNKDADLYTSRVMLSSQVPLEPPLLFLLEEYKNYLDAANMSM**X₁X₂X₃X₄**HS DPARRGELSVCDSISEWVTAADKKTAVDMSGGTVTVLEKVPVSKGQLKQYFYETKCN PMGYTKEGCRGIDKRHWNSQCRTTQSYVRALTMDSKKRIGWRFIRIDTSCVCTLTIKR GR, wherein X1, at position 108, X2, at position 109 and X3, at position 110, are selected from non-basic amino acids and histidine and X4, at position 111, is selected from Arginine and Lysine.

Preferably, X1, X2 and X3 are independently selected from the group consisting of Alanine, Asparagine, Aspartic Acid, Cysteine, Glutamine, Glutamic Acid, Glycine, Isoleucine, Leucine, Methionine, Phenylalanine, Proline, Serine, Threonine, Tryptophan, Tyrosine, Valine and Histidine (SEQ ID NO: 5).

More preferably, X1, X2 and X3 are independently selected from the group consisting of Valine, Alanine, Glycine, Serine, Threonine, Tyrosine, Asparagine, Aspartic Acid, Glutamine, Glutamic Acid, Methionine and Histidine (SEQ ID NO: 6).

In one preferred embodiment, X1 and X3 are independently selected from Alanine and Valine (SEQ ID NO: 7); in another preferred embodiment, X2 is selected from Valine and Serine (SEQ ID NO: 8); in a further preferred embodiment, X4 is arginine (SEQ ID NO: 9).

According to a preferred embodiment, X1 and X3 are independently selected from Alanine and Valine, X2 is selected from Valine and Serine and X4 is Arginine (SEQ ID NO: 10).

A particularly preferred proBDNF mutein according to the invention is the mutein of SEQ ID NO: 4, corresponding to a mutein having the amino acid sequence of SEQ ID NO: 3, wherein X1 is Valine, X2 is Serine, X3 is Alanine and X4 is Arginine.

A further object of the present invention is a nucleic acid encoding for a human proBDNF mutein as described herein.

A further object of the present invention is a method of recombinantly producing a human BDNF, comprising:
(i) providing a solution of a human proBDNF mutein according to the first object of the invention,
(ii) purifying the human proBDNF mutein from the solution provided in step (i), thereby obtaining a solution of purified human proBDNF mutein, and
(iii) cleaving the human proBDNF mutein in said solution to obtain human BDNF.

Preferably, said method of recombinantly producing a human BDNF comprises:
(i) providing a solution of a human proBDNF mutein according to the first object of the invention, wherein said human proBDNF mutein is correctly folded, by expressing the human proBDNF mutein according to the first object of the invention in a host cell cultured in a liquid culture medium and isolating the human proBDNF mutein from the host cell;
(ii) purifying the human proBDNF mutein from the solution provided in step (i) via chromatographic purification, preferably using an eluent solution comprising urea, more preferably an eluent solution comprising citric acid and urea, thereby obtaining a solution of purified human proBDNF mutein; and
(iii) cleaving the prosequence of the human proBDNF mutein in said solution, to obtain a biologically active human BDNF; preferably, before said cleaving, the pH of the solution obtained in step (ii) is adjusted to a pH value comprised between 5.8 and 9.

Preferably, step (i) of said method of recombinantly producing a human BDNF comprises the following steps:
a) providing a nucleic acid encoding a human proBDNF mutein according to the first object of the invention;
b) introducing said nucleic acid into an expression vector;
c) introducing said expression vector into host cells;
d) growing said host cells in a suitable liquid culture medium, wherein said host cells express the human proBDNF mutein according to the first object of the invention in form of inclusion bodies;
e) lysing the host cells and isolating the human proBDNF mutein inclusion bodies produced by the host cells in step d);
f) dissolving the inclusion bodies in a denaturing solution, thereby obtaining a solution of denatured human proBDNF mutein;
g) diluting the solution of denatured human proBDNF mutein into a refolding solution, so that the denatured human proBDNF mutein assumes a correctly folded conformation, thereby obtaining a solution of the correctly folded human proBDNF mutein.

Preferably, the nucleic acid provided in step a) is DNA.

Preferably, the nucleic acid provided in step a) has a codon-optimized sequence, more preferably a codon-optimized DNA sequence, for efficient expression in the host cell, preferably in *E. coli.*

Preferably, the expression vector of step b) is selected from pET28a, pBR322, pMAL, pUC19 and all derivatives, more preferably it is pET28a. However, all suitable expression vectors known in the art may be used for this purpose.

Preferably, said host cells of step c) are prokaryotic host cells.

Suitable prokaryotic host cells that can be used according to the present invention are well known in the art and include, but are not limited to, prokaryotic cells such as bacteria, for example, *E. coli, Bacillus sp.,* and *Salmonella,* which can be transformed with, for example, plasmid DNA, recombinant bacteriophage DNA, or cosmid DNA expression vectors containing the nucleic acid of the invention; kits for such expression systems are commercially available.

According to a preferred embodiment, said host cells of step c) are *E. coli,* preferably selected from *E. coli* BL21 (DE3), JM 108/109 (K12), JM106, JM83 and TBI. Any other *E. coli* strain suitable for expression of recombinant proteins could be used.

In step c) introducing said expression vector into the host cells may be carried out by any method known in the art. Preferably, according to the present invention, introducing said expression vector into host cells comprises transforming the expression vector into the host cells, preferably *E*. *coli,* by temperature shock.

Preferably, in step d) said suitable liquid culture medium in which the host cells are grown is Terrific Broth (TB).

Preferably, in step d) said liquid culture medium contains kanamycin, preferably at the concentration of 30 ug/ml.

Preferably, in step d) said liquid culture medium contains a compound that induces the expression of the recombinant protein, preferably isopropyl β- d-1-thiogalactopyranoside (IPTG).

Preferably, in step d) a compound that induces the expression of the recombinant protein, preferably isopropyl β- d-1-thiogalactopyranoside (IPTG), is added when an optical density of 1 measured at 600 nm (OD₆₀₀=1) is reached.

Preferably, the inclusion bodies obtained in step d) may also contain proteins different from the human proBDNF mutein of the invention. Preferably, said inclusion bodies contain at least 60 wt.%, at least 70 wt.%, at least 80 wt.% or at least 90 wt% of human proBDNF mutein according to the invention, based on the total amount of protein present in the inclusion bodies.

In step e) lysing the host cells may be performed by conventional methods, e.g. by means of high pressure homogenization, sonification or lysozyme (Rudolph, R., et al. (1997); Folding proteins. In: Creighton, T. E. (ed.): Protein Function: A Practical Approach. Oxford University Press, pp. 57-99).

Preferably, in step e) lysing the host cells is carried out by high pressure homogenization in a lysis buffer. Preferably, said lysis buffer comprises, preferably consists of, 0.1 M Tris Buffer at pH 7, containing 0.025 M EDTA.

Preferably, in step e) isolating the human proBDNF mutein inclusion bodies is carried out by sequential centrifugations of the host cell lysis product.

Preferably, in step f), the denaturing solution comprises the following components:
i. Guanidinium-HCl, 1-8 M, preferably 3-6 M, more preferably 4 M, Cysteine, 1-100 mM, preferably 5 mM,
ii. Tris, 0.001 -1M, preferably 0.1 M,
iii. EDTA, 1-50 mM, preferably 10 mM,
and has a pH between 7 and 10, preferably of 8.

After the solubilization of the human proBDNF mutein, the protein is refolded so as to obtain a correctly folded human proBDNF mutein. For the refolding process it is important to minimize the competing reactions of misfolding and aggregation. To prevent aggregation the refolding is performed at very low protein concentrations (about 250 µg per ml) because aggregation of the protein is predominant at high protein concentrations. Preferably, in step g), the refolding solution comprises:
i. a chaperone, preferably Arginine, preferably at a concentration of 0.5-1.0 M, more preferably 0.75 M,
ii. a metal chelator, preferably EDTA, preferably at a concentration of 1-10 mM, more preferably 5 mM,
iii. a redox shuffling system, preferably selected from a combination of L-Cystine and L-Cysteine and a combination of oxidized glutathione and reduced glutathione, more preferably selected from a combination of 1 mM L-Cystine and 5 mM L- Cysteine and a combination of 1 mM GSSG (oxidized glutathione) and 5 mM GSH (reduced glutathione); and has a pH between 8 and 11.

Alternative redox shuffling systems such as Cystamin/Cysteamin could be used. Preferably, 360 ml of the solution of denatured human proBDNF mutein are diluted into 6 L of refolding solution as disclosed above. Preferably, the concentration of Guanidine in the final solution obtained after dilution in the refolding solution is at most 0.3 M. According to the invention, a chaperone is a compound which promotes the folding of proteins. Such compounds are known to the person skilled in the art. They can assist the folding in various ways. Arginine is a preferred chaperone according to the invention. Arginine destabilizes incorrectly folded intermediates, so that these are at least partly unfolded (from a thermodynamic dead-end) and therefore can be correctly folded again. Preferably, step g) further comprises a step of adjusting the pH of the final solution where refolding of the human proBDNF mutein occurs to a value between 8.5 and 9.5, more preferably to a value between 8.8 and 9.2, even more preferably to a value of 9.1.

By carrying out the method according to the invention with the denaturation and subsequent refolding, an aqueous solution of the correctly folded human proBDNF mutein is obtained.

Preferably, in step (ii) of said method of recombinantly producing a human BDNF the solution of the purified human proBDNF mutein obtained comprises urea, more preferably, comprises citric acid and urea.

Preferably, in said solution urea has a concentration below 2 M, more preferably a concentration of 1 M. Preferably, in said solution citric acid has a concentration between 5 mM and 100 mM, more preferably a concentration of 50 mM. Preferably, said solution has a pH ranging from 3 and 5.5, preferably a pH of 4.

Preferably, step (ii) comprises purifying the human proBDNF mutein from the solution provided in step (i) via chromatographic purification.

More preferably, in said chromatographic purification, an eluent solution comprising urea, more preferably citric acid and urea is used. According to this embodiment, the eluate obtained in the chromatographic purification can be directly used in step (iii). Preferably, in said eluent solution urea has a concentration below 2 M, more preferably a concentration of 1 M. Preferably, in said eluent solution citric acid has a concentration between 5 mM and 100 mM, more preferably a concentration of 50 mM. Preferably, said eluent solution has a pH ranging from 3 and 5.5, preferably a pH of 4.

Preferably, said chromatographic purification is carried out by mixed mode chromatography.

In an embodiment, the pH of the solution provided in step (i) is adjusted to 8 before loading on the chromatographic column.

In an alternative embodiment, the pH of the solution provided in step (i) is not modified before loading on the chromatographic column, so the pH is comprised between 8 and 11, preferably it is comprised between 8.5 and 9.5, more preferably it is comprised between 8.8 and 9.2, even more preferably it is 9.1.

The most preferred column used in the chromatographic purification of step ii) is a column with a synthetic affinity ligand, preferably 4-mercapto-ethyl-pyridine (MEP Hypercell; Sartorius). Advantages of this medium are that the binding is independent of the ionic strength, salt stacking is not necessary and higher flow rates to fasten the process are possible. Further, the elution is done by a pH-shift.

Other mixed mode material columns are known and could be used. For example, but not limited to, MEP (Sartorius; affinity ligand is 4-Mercapto ethyl pyridine), HEA (Sartorius; affinity ligand: Hexylamino), PPA (Sartorius, affinity ligand: Phenylpropylamino), MBI (Sartorius; affinity ligand: 2-Mercapto-5benzamidazole sulfo acid), Capto MMC (GEHC), Capto adhere (GEHC; affinity ligand: N-benzyl-N-methyl ethanolamine), CHT hydroxyapatite (BioRad), CHT fluoroapatide). The MEP, HEA, PPA, and MBI columns have a hydrophobic binding, where Capto MMC is a cation exchanger with mixed mode functionality and Capto adhere is an anion exchanger with mixed mode functionality. The BioRad columns are Ion exchange columns with hydrophobic components.

Preferably, in step (iii) of said method of recombinantly producing a human BDNF said cleaving comprises adjusting the pH of the solution of the purified human proBDNF mutein obtained in step (ii) to a pH value between 5.8 and 9, more preferably a pH value between 5.8 and 8, more preferably a pH value between 5.8 and 7, more preferably a pH value between 5.8 and 6.5, even more preferably a pH value of 6, and cleaving the human proBDNF mutein in said solution to obtain biologically active human BDNF.

Preferably, said cleaving the human proBDNF mutein is carried out by a trypsin-like protease, more preferably by trypsin.

Proteases having trypsin-like substrate specificity cleave the protein without digesting the active portion of the protein molecule. Trypsin-like proteases cleave peptide bonds following a positively charged amino acid such as Arginine or Lysine. Preferably, Trypsin is used for the cleavage of the pro-sequence but other proteases could be used instead. It is noted that cleavage is not restricted to trypsin itself, but may involve other proteases having trypsin-like substrates as well. Generally, the ratio of human proBDNF mutein to trypsin (or other protease) is appropriately adjusted so that the correctly folded, mature human BDNF will not be cleaved by this protease. In contrast, denatured proteins as well as folding intermediates expose sequences which are susceptible to an attack by the protease.

Preferably for the cleavage of the human proBDNF mutein to human BDNF, the ratio of trypsin-like protease, preferably trypsin, to human proBDNF mutein is between 1 : 20000 - 1 : 30000 w/w, more preferably between 1 : 23000 - 1 : 27000 w/w, most preferred is a ratio of 1 : 25000 w/w. In a preferred embodiment, the cleavage occurs for more than 13 hours at room temperature, preferably for a time comprised between 13 hours and 17 hours, most preferably for 15 hours or 16 hours.

Preferably, step (iii) further comprises the step of inhibiting the activity of the trypsin-like protease.

Preferably, said inhibiting is carried out by adjusting the pH of the solution of human BDNF obtained in step (iii) to a value between 4.5 and 5.5, preferably 5.

Preferably, said inhibiting is carried out by diluting the solution obtained in step (iii) with a solution of arginine, citric acid and urea, so as to obtain a final solution with a pH value between 4.5 and 5.5 and a concentration of arginine between 0.1 M and 0.2 M, more preferably a concentration of 0.125 M. Preferably, said solution comprises: 1M arginine, 50 mM citric acid and 1 M urea, and is added to the solution of step (iii) with a dilution ratio 1/8 in order to obtain a drop in pH around 5.2 and a final arginine concentration of 0.125 M. In these conditions Arginine has the advantages of:
i) naturally dropping the pH, ii) acting as a chaperon and prevent protein aggregation iii) inhibiting trypsin activity as substrate competitor, and iv) being compatible with the chromatography of the following step (iv) of purification of human BDNF, when present. According to a preferred embodiment, the method of the present invention further comprises step (iv) of purifying the human BDNF obtained in step (iii).

Preferably, said purifying in step (iv) comprises separating trypsin and product-related impurities of the tryptic digestion from BDNF.

Preferably, said purifying also reduces Host cell proteins (HCPs), Endotoxins, and DNA impurities.

Any methods known in the art for protein purification can be used in step (iv). Preferably, said purifying in step (iv) comprises one or more chromatographic purification steps.

Preferably, Sepharose columns, more preferably SP Sepharose High Performance, Phenyl Sepharose 6 Fast Flow or Q Sepharose Fast Flow columns, are used in said one or more chromatographic purification steps.

Preferably step (iv) comprises a chromatographic purification of the human BDNF on SP Sepharose High Performance column, more preferably followed by a further purification on a Phenyl Sepharose Fast Flow low substitution column.

The final product BDNF produced from a human proBDNF mutein may be analyzed regarding its purity by SDS-PAGE, rp-HPLC, SE-HPLC, IEX-HPLC and RP-UPLC. Preferably, the human BDNF obtained by the method according to the present invention has a purity, measured by RP-UPLC, of at least 94%, preferably at least 95%, more preferably at least 96%, more preferably at least 97%, more preferably at least 98%, even more preferably at least 99%.

This high level of purity is achievable due to the specific features of the method according to the present invention identified by the inventors.

The present inventors have found that the cleavage of the pro-sequence from the human proBDNF mutein according to the invention by trypsin-like proteases to produce human BDNF has a higher efficiency compared to the cleavage of the pro-sequence from the wild-type proBDNF. Therefore, lower amounts of enzyme are necessary in order to obtain the final human BDNF protein, resulting in a reduced production of cleveage by-products and an easier purification.

Furthermore, as will also be described more in details in the experimental section below, the inventors have found that the use of a solution comprising urea as cleavage buffer is advantageous, as in this condition the protein is correctly cut, and no precipitation is observed.

Furthermore, the inventors have found that, when the purification of step (ii) is carried out by a chromatographic purification using as eluent a solution comprising urea and citric acid, step (iii) of cleavage of the human proBDNF mutein and step (iv) of purification of BDNF produced can all be carried out in the same solution eluted from the chromatographic column of step (ii), by simply adjusting the pH to the value needed in the specific process step with addition of acid(s) or base(s) to the solution containing citric acid and urea. This represents a considerable advantage, since the operational flow is simplified and the process requires the use of less materials, which allows to facilitate the further purification step and to achieve a final product with high purity. In particular, the use of an eluent comprising citric acid and urea in step (ii) results in avoiding the need to change buffer by tangential flow filtration at the end of the purification step and before the cleavage step. Furthermore, the association of citric acid with urea not only allows to easily modify the pH of the solution, but also prevents protein aggregation and, surprisingly, does not interfere with trypsin activity.

A further object of the present invention is the use of a human proBDNF mutein according to any one of the above-described embodiments of the first object of the invention in a method of producing human BDNF.

A further object of the present invention is a human BDNF obtainable, or obtained, by the method according to the present invention.

A further object of the present invention is a human BDNF having a purity, measured by RP-UPLC, of at least 94%, preferably at least 95%, more preferably at least 96%, more preferably at least 97%, more preferably at least 98%, even more preferably at least 99%. A further object of the present invention is a human BDNF composition containing the peptide impurity having sequence: 
GELSVCDSISEWVTAADKKTAVDMSGGTVTVLEKVPVSKGQLKQYFYETKCNPMGYT
KEGCRGIDKRHWNSQCRTTQSYVRALTMDSKKRIGWRFIRIDTSCVCTLTIKRGR
(SEQ ID NO: 22)
 in an amount equal or below 5%, preferably in an amount equal or below 4%.

A further object of the present invention is a pharmaceutical composition comprising the human BDNF as above described and at least one pharmaceutically acceptable ingredient.

In an embodiment, said pharmaceutically acceptable ingredient may be a diluent, a carrier, a filler, a salt, a buffer, a stabilizing agent, a penetration enhancer. Techniques for the formulation of pharmaceutical compositions of the present invention may be found in standard textbooks such as "Remington's Pharmaceutical Sciences", Mack Publishing Co., Easton, PA, recent edition.

The invention will be further described in the following examples, which do not limit the scope of the invention as defined in the claims.

### EXAMPLES

### Example 1 - Cloning of the DNA encoding proBDNF mutein into an expression vector and insertion into E. coli

A synthetic gene corresponding to a human proBDNF mutein of SEQ ID NO: 4 was prepared, with triplet codons optimized for their expression in *Escherichia coli.* For cloning, a Ncol restriction site was engineered in the initial methionine residue (CCATGG) and a Xhol restriction site was inserted after the last stop codon.

After digestion by Ncol and Xhol restriction enzymes, the corresponding DNA fragment encoding proBDNF ORF was introduced into a pET28a expression vector (Novagen) cut by the same enzymes.

The expression vector obtained was transformed into *Escherichia coli* BL21 (DE3) strain by temperature shock according to supplier instructions (BL21 (DE3) Chemi Competent cells ref 156-3003; BioRad).

### Example 2 - Fermentation and induction of expression of the protein

A single colony of the recombinant *E*. *coli* strain was inoculated in 30 ml of Terrific Broth (TB) medium (Sigma Aldrich) containing 30 µg/ml of kanamycin, which was then incubated under agitation at 37°C over night. The day after, 300 ml of fresh TB, containing 30 µg/ml kanamycin, were inoculated with 3 ml of the overnight culture, and when OD₆₀₀ₙₘ reached 1, 1mM Isopropyl β- d-1-thiogalactopyranoside (IPTG) was added to the medium to induce the expression of the recombinant protein and the culture was continued on for three further hours.

To monitor the over-expression of the target protein during the fermentation process, samples were analyzed by means of SDS-PAGE before and after induction.

As human proBDNF mutein possesses three intrachain disulfide bridges, the protein was accumulated in the cytoplasm of the cells in form of inclusion bodies (iBs).

### Example 3 - Isolation and solubilization of inclusion bodies

The cells were harvested by centrifugation, resuspended at 1.5 weight/volume in Lysis buffer (0.1 M tris, 0.025 M EDTA, pH 7.0) and homogenized with a NS1001L2K Niro Soavi high-pressure homogenizer for four cycles at 800 bars. The homogenate obtained was diluted 1:0.5 with a preparation of Brij 35 (60gr/L) and stirred for 30 minutes.

The pellets corresponding to inclusion bodies were isolated by centrifugation (8500 rpm, Sorval GS3 rotor), washed twice with Tris 0.1 M pH 7.0 at 0.4 to 1 weight/volume. The mixture was centrifuged (7500 rpm, Sorval GS3 rotor) and the pellets dissolved in guanidine solution to reach 4 M final concentration. In particular, the following guanidine solution was used:
i. Guanidinium-HCl 4 M, Cysteine 5 mM,
ii. Tris 0.1 M,
iii. EDTA 10 mM,
iv. pH 8.0

### Example 4 - Refolding of the proBDNF mutein

To prepare the correctly folded human proBDNF mutein according to the invention, 360 mL of the solubilized material obtained according to Example 3 was diluted into 6 L of a refolding solution having the following composition: 0.75 M arginine-HCl, 5 mM EDTA, 5 mM cysteine-HCl and 1 mM cystine, and maintained at a temperature between 0 and 10°C, wherein the human proBDNF mutein assumes a correctly folded conformation. The concentration of guanidine after dilution into the refolding solution was at most 0.3 M. The pH of the refolding solution was adjusted to 9.1 with sodium hydroxide.

The performance of the refolding reaction was analyzed by RP-HPLC and was estimated to be 55% of the initially solubilized protein.

### Example 5 - Purification of the human proBDNF mutein from the refolding solution

In order to purify the human proBDNF mutein from the refolding solution, a hydrophobic induced charge chromatography was carried out using a column with the synthetic affinity ligand 4-mercapto-ethyl-pyridine (MEP). Before loading the solution with the refolded protein, the column was equilibrated with five column volumes of 0.75 M arginine-HCl, 5 mM EDTA pH 9.1.

Two different experimental settings, named **5a** and **5b,** were tested for elution of the protein from the column, which are summarized below.
- Setting **5a →** in this setting, the refolded human proBDNF mutein was loaded after filtration on the MEP column, the column was washed with 0.1 M Tris pH 8.0, and the protein eluted in 0.75 M arginine-HCl, 0.05 M acetic acid buffer at pH 4.0. The eluted fraction containing the protein was then buffer exchanged by tangential flow filtration, on a cassette having a cutoff of 10 kDa, with 6 successive diafiltrations performed with 0.025 M sodium phosphate pH 6.5 in order to remove arginine and favor the subsequent enzymatic cleavage of the human proBDNF mutein, which will be discussed in the following example.
- Setting **5b →** in this setting, the refolded human proBDNF mutein was loaded directly at pH 9.1 in the refolding solution, the column was washed with Tris Buffer at pH 8.0 and the protein was eluted with a solution containing 50 mM citric acid at pH 4.0 and urea 1 M.

Without being bound to a theory or principle, the inventors have found that setting **5b,** where the human proBDNF mutein is eluted with a solution containing citric acid and urea, is advantageous over setting **5a,** where the human proBDNF mutein is eluted in arginine buffer which is then exchanged to phosphate buffer, in view of the reasons that will be explained in the following example.

### Example 6 - Cleavage of the human proBDNF mutein to obtain mature BDNF

Once eluted from the MEP column, the purified human proBDNF mutein was cleaved to form mature BDNF. Trypsin is the preferred enzyme to cleave the human proBDNF mutein to obtain mature BDNF, since it cuts after Arginine (R) or Lysine (K) and is commercially available in GMP grade. Thus, the purified protein solution obtained according to example 5 was incubated with trypsin. The pH of the protein solution was adjusted to a value of 6.5 or 6.0 (see Table 1 below) to favor enzymatic cleavage of the human proBDNF mutein by trypsin.

The present inventors tested several experimental settings for the cleavage step, named **6a** to **6g**, whose conditions are summarized in Table 1 below and discussed in the afterwards.

**Table 1**

| **Cleavage Setting** | **Trypsin/ ProBDNF mutein ratio (w/w)** | **Cleavage buffer** | **Additive in cleavage buffer** | **Time of digestion** |
|---|---|---|---|---|
| **6a** | 1/7500 | 25 mM phosphate pH 6.5 | / | 16 hours |
| **6b** | 1/7500 | | 0.5 M arginine | |
| **6c** | 1/7500 | | 10% glycerol | |
| **6d** | 1/7500 | | 0.1 % Tween 20 | |
| **6e** | 1/7500 | | 1% threalose | |
| **6f** | 1/7500 | 50 mM Citric acid pH 6.5 | 1 M urea | 2 hours |
| **6g** | 1/25000 | 50 mM Citric acid pH 6.0 | 1 M urea | 15 hours |

In order to purify the human proBDNF mutein and subsequently cleave it, the inventors first tried the purification setting **5a** described in Example 5, followed by each one of the cleavage settings **6a** to **6e** described in Table 1.

When cleavage setting **6a** was carried out, the formation of a heavy precipitate was observed as digestion was going on during incubation.

Without being bound to a theory or principle, the inventors hypothesized that this precipitation was due to the peptides generated by trypsin activity, which aggregated as digestion was proceeding. As the solubilized material aggregated and precipitated, the soluble rhBDNF formed was taken with it due to a carry over phenomenon.

As Trypsin cuts after lysine (K) and arginine (R) residues, looking at the pro-protein sequence all the following peptides could be generated during digestion.
1. MAPMK (SEQ ID NO: 11)
2. EANIR (SEQ ID NO: 12)
3. GQGGLAYPGVR (SEQ ID NO: 13)
4. THGTLESVNGPK (SEQ ID NO: 14)
5. AGSR (SEQ ID NO: 15)
6. GLTSLADTFEHVIEELLDEDQK (SEQ ID NO: 16)
7. VR
8. PNEENNK (SEQ ID NO: 17)
9. DADLYTSR (SEQ ID NO: 18)
10. VMLSSQVPLEPPLLFLLEEYK (SEQ ID NO: 19)
11. NYLDAANMSMVSAR (SEQ ID NO: 20)

Also, a further hepta peptide, corresponding to the first seven amino acids of the BDNF with the following sequence: HSDPARR (SEQ ID NO: 21), could be generated during digestion. In fact, this peptide bearing two arginine at its carboxy terminus is accessible during trypsin digestion and its cleavage generates a truncated form of BDNF, which is named "hyperdigested" in the following, having the following sequence: GELSVCDSISEWVTAADKKTAVDMSGGTVTVLEKVPVSKGQLKQYFYETKCNPMGYT KEGCRGIDKRHWNSQCRTTQSYVRALTMDSKKRIGWRFIRIDTSCVCTLTIKRGR (SEQ ID NO: 22). As it has been demonstrated that those 7 amino acids (HSDPARR) are responsible of the main interaction between BDNF and its receptor TrkB, it can be hypothesized that this truncated BDNF form is inactive. Therefore, during trypsin cleavage not only the precipitation of the product must be controlled, but also the generation of the hyperdigested BDNF form, which needs to be removed by the successive chromatographies together with the peptides corresponding to the digestion of the pro part.

To try to maintain the protein in solution, each one of cleavage settings **6b** to **6e** reported in Table 1 above was tested instead of cleavage setting **6a** after purification setting **5a** described in Example 5 was carried out. Those settings involved adding various materials to the protein solution obtained after procedure **5a** to try to maintain the protein in solution as digestion was proceeding. However, as enzymatic digestion was going on, the protein precipitated even in presence of those additives.

The inventors then decided to add urea (1 M) in the cleavage buffer.

Without being bound to a theory or principle, the inventors hypothesized that urea may act as disaggregating agent and prevent precipitation of the protein from the solution. However, the use of urea could also have been detrimental for producing the desired BDNF, since it may denature the proBDNF mutein and render accessible new cleavage sites that were previously buried inside the refolded protein.

Surprisingly, the present inventors have found that, when adding urea in the cleavage buffer, the protein was correctly cut in only two hours and no precipitation was observed. Based on these results, it was decided that the preferred cleavage setting involved adding urea 1 M in the cleavage buffer.

Then, to further simplify the process, the inventors tested a different purification setting, namely setting **5b** described in Example 5 above, followed by cleavage setting **6f** described in Table 1 above.

Without being bound to a theory or principle, the inventors have found that the combination of the conditions of purification setting **5b** and of cleavage setting **6f** is advantageous, as it leads to a notable simplification of the operational flow of the method according to the present invention.

Indeed, setting **5b** disclosed in Example 5, where the human proBDNF mutein is eluted with a solution containing citric acid and urea, is advantageous over setting **5a,** where the human proBDNF mutein is eluted in arginine buffer which is then exchanged to phosphate buffer, in view of the following.

When setting **5b** is used instead of setting **5a,** the eluate containing purified human proBDNF mutein that is eluted from the MEP column can be used directly as buffer solution for the subsequent cleavage by trypsin of human proBDNF mutein in mature BDNF and successive ion exchange chromatography for purifying the mature BDNF, by adjusting the pH to the value needed in the specific process step with addition of acid(s) or base(s) to the solution containing citric acid. This is possible because citric acid has 3 pKa values at 25 °C (5.21, 4.28 and 2.92) and, therefore, is able to cover, in the present process, all the needed buffer range going from pH 4.0 of the MEP elution up to pH 6.0 or 6.5 for enzymatic digestion.

The presence of urea in the elution solution of setting **5b** is also advantageous, not only because it prevents aggregation and precipitation during the subsequent cleavage step of the human proBDNF mutein, but also because it prevents from using Arginine during the purification step of human proBDNF mutein, thus avoiding the need of carrying out tangential flow filtration to formulate the protein solution in order to digest it with trypsin, as in setting **5a.** Indeed, Arginine is well known to be helpful in maintaining proteins in solution, and therefore is useful to counteract the natural tendency to aggregate of proBDNF, but is also a substrate of trypsin, so it has to be removed before cleaving the human proBDNF mutein into BDNF. Its removal complicates the operational flow of the process, and this drawback is solved by using the eluent according to the present invention, which does not involve the use of Arginine. Not using Arginine in the step of purification of the human proBDNF mutein also allows to increase the efficiency of the subsequent cleavage of the human proBDNF mutein by trypsin. As a consequence, less trypsin needs to be used, which greatly simplifies the final purification of mature BDNF obtained.

Based on all the above, the association of citric acid with urea is the preferred solution to elute the MEP column, perform enzymatic digestion and load digested material on the further Cation Exchange Chromatography (CEX) column, all in one compatible buffer. The parameters of the cleavage setting **6f** were finally slightly modified to increase the digestion time so as to allow the possibility of carrying out some in-process control. Cleavage setting **6g** was therefore tested. In this specific setting, the pH of the solution was adjusted to 6.0, a value at which the activity of trypsin is lower than at 6.5, and the ratio of trypsin/proBDNF mutein was changed from 1/7500 to 1/25000. The temperature was maintained at 22 °C for all the reaction time.

This trypsin/proBDNF mutein ratio is surprisingly very low. Since trypsin needs to be removed from the final product, having a lower amount of trypsin is advantageous and results in a simplification of the purification process. In fact, the isoelectric point of trypsin is 10.5, which means it will bind to the SP Sepharose column that is used after cleavage to purify the BDNF from the fragments generated during digestion (see Example 7 below). Therefore, the less trypsin used during the cleavage step, the better, both in terms of obtaining higher cleavage specificity and easier purification of mature BDNF formed. Different digestion times were tested for the cleavage of human proBDNF mutein, namely 13 hours, 14 hours, 15 hours, 16 hours, and 17 hours. These digestion times were long enough to allow introducing some in-process control. The results are reported in Table 2 below.

**Table 2**

| **Digestion time (h)** | **ProBDNF mutein (µg/µl)** | **BDNF (µg/µl)** | **Hyper digested (%)** |
|---|---|---|---|
| 0 | 569.9 | | |
| 13 | 17.0 | 91.7 | 22.2 |
| 14 | 15.1 | 140.3 | 33.3 |
| 15 | 14.6 | 144.6 | 38.3 |
| 16 | 12.6 | 161.0 | 38.3 |
| 17 | 7.8 | 159.6 | 46.7 |

The temperature of the reaction was maintained at 22 °C for all digestion times used.

As can be seen, all the digestion time tested (i.e., 13 hours and up) resulted in the production of BDNF from the human proBDNF mutein with satisfactory yields and with low formation of the hyperdigested form. In particular, the human proBDNF mutein disappeared almost completely at 15 hours (remaining 2.56% of the initial human proBDNF mutein) and the hyperdigested form was around 38%, an amount that can be removed by the successive chromatographies. Those parameters were also valid for 16 hours, which leave sufficient margin of time to process the solution.

Based on these results, purification setting **5b** followed by cleavage setting **6g** were selected as the preferred conditions for the purification of refolded human proBDNF mutein and subsequent cleavage into mature BDNF, respectively.

### Example 7 - Purification of active BDNF

After the tryptic digestion was completed, the pH of the cleavage solution containing BDNF was adjusted to a value of 5.0 by addition of a solution of 1 M arginine, 50 mM citric acid and 1 M urea at a ratio 1/8 to inhibit the activity of trypsin, and then the solution was loaded on a SP Sepharose high Performance column to deplete Trypsin, by-products of the cleavage and further impurities. The solution was filtered before loading in order to protect the column.

The column was previously equilibrated with 50 mM citric acid, 1 M urea pH 5.0. The column was then washed with 25 mM phosphate buffer pH 6.5 and the protein was then eluted in the same buffer increasing the salt concentration with step gradient.

During elution, the different peaks have been fractionated, with fractions 9 and 10 corresponding to the main peak. All fractions have then been analyzed by RP-UPLC and the results are reported in Table 3 below.

**Table 3**

| **Fraction Sample** | **Area % Hyper vs BDNF** | **Cone. BDNF tot µg/mL** |
|---|---|---|
| **Load** | **33.0** | **116.8** |
| 1 | 0.0 | 3.1 |
| 2 | 0.0 | 1.1 |
| 3 | 30.5 | 6.6 |
| 4 | 49.2 | 10.7 |
| 5 | 31.6 | 21.2 |
| 6 | 36.1 | 23.9 |
| 7 | 22.2 | 127.3 |
| 8 | 34.3 | 98.9 |
| **9** | **9.3** | **246.7** |
| **10** | **10.9** | **287.3** |
| 11 | 44.6 | 78.3 |
| 12 | 88.6 | 88.9 |

Results indicate that the BDNF protein is eluted in the main peak with a percentage of hyperdigested form dropping from 33%, as present in the loaded material, to around 10%. Fractions 9 and 10 were pooled and the resulting solution was adjusted to 2 M NaCl concentration.

At this point, the solution was filtered and loaded on a Phenyl Sepharose Fast Flow low substitution column. The column was preequilibrated with phosphate buffer pH 6.5, 2 M NaCl and the protein eluted by decreasing the salt concentration.

Protein was eluted as a single fraction corresponding to 5 column volumes.

The analysis carried out on this fraction confirmed that the protein was pure at 94%.

### Example 8 - Test for the biological activity of BDNF

The biological activity of the recombinant human BDNF obtained according to the method disclosed above was tested by using rat C6 glioma cells that express high levels of BDNF TrkB receptor (Xiong J. et al., Oncology Letters 2015; 10:223-227). It was demonstrated that through this receptor, mature BDNF promotes C6 cell growth and survival in a dosedependent manner (Xiong J. et al., Oncology Reports 2013; 30(6):2719-2724). C6 cells were plated in 96-well microplates at a density of 5000 cells/well and treated with rhBDNF concentration ranging from 0.23 to 9 µg/mL. After 48h incubation, the CellTiter 96^{®} AQᵤₑₒᵤₛ One Solution Cell proliferation Assay (MTS assay) was perfomed in order to determine the biological activity of rhBDNF from the number of viable C6 cells.

The EC₅₀, corresponding to the concentration of rhBDNF required to induce 50% proliferation of the cells, was determined for rhBDNF produced according to the method disclosed above by using the GraphPad statistical software and a mean value of about 3 µg/mL (0.11 µM) was obtained (Figure 1).

## Claims

1. A human proBDNF mutein having the following amino acid sequence: 
MAPMKEANIRGQGGLAYPGVRTHGTLESVNGPKAGSRGLTSLADTFEHVIEELLDE
DQKVRPNEENNKDADLYTSRVMLSSQVPLEPPLLFLLEEYKNYLDAANMSMX1X2X
3X4HSDPARRGELSVCDSISEWVTAADKKTAVDMSGGTVTVLEKVPVSKGQLKQYF
YETKCNPMGYTKEGCRGIDKRHWNSQCRTTQSYVRALTMDSKKRIGWRFIRIDTSC
VCTLTIKRGR (SEQ ID NO:3),
 wherein X1, X2 and X3 are selected from non-basic amino acids and histidine and X4 is selected from Arginine and Lysine.

2. The human proBDNF mutein according to claim 1, wherein X1, X2 and X3 are independently selected from the group consisting of Alanine, Asparagine, Aspartic Acid, Cysteine, Glutamine, Glutamic Acid, Glycine, Isoleucine, Leucine, Methionine, Phenylalanine, Proline, Serine, Threonine, Tryptophan, Tyrosine, Valine and Histidine; (SEQ ID NO:5) preferably from the group consisting of Valine, Alanine, Glycine, Serine, Threonine, Tyrosine, Asparagine, Aspartic Acid, Glutamine, Glutamic Acid, Methionine and Histidine (SEQ ID NO:6).

3. The human proBDNF mutein according to claims 1 or 2, wherein, independently from each other X1 and X3 are selected from Alanine and Valine; X2 is selected from Valine and Serine; X4 is Arginine (SEQ ID NO:10).

4. The human proBDNF mutein according to any one of claims 1-3, wherein X1 is Valine, X2 is Serine, X3 is Alanine and X4 is Arginine (SEQ ID NO:4).

5. A nucleic acid encoding the human proBDNF mutein according to any one of claims 1-4.

6. A method of producing a human BDNF comprising the steps of:
(i) providing a solution of a human proBDNF mutein as defined in any one of claims 1-4;
(ii) purifying the human proBDNF mutein from the solution provided in step (i), thereby obtaining a solution of purified human proBDNF mutein, and
(iii) cleaving the human proBDNF mutein in said solution to obtain human BDNF.

7. The method according to claim 6, wherein step (i) comprises the following steps:
a) providing a nucleic acid encoding a human proBDNF mutein as defined in any one of claims 1 to 4;
b) introducing said nucleic acid into an expression vector;
c) introducing said expression vector into host cells;
d) growing said host cells in a suitable liquid culture medium, wherein said host cells express the human proBDNF mutein in form of inclusion bodies;
e) lysing the host cells and isolating the human proBDNF mutein inclusion bodies produced by the host cells in step d);
f) dissolving the inclusion bodies in a denaturing solution, thereby obtaining a solution of denatured human proBDNF mutein;
g) diluting the solution of denatured human proBDNF mutein into a refolding solution, so that the denatured human proBDNF mutein assumes a correctly folded conformation, thereby obtaining a solution of the correctly folded human proBDNF mutein.

8. The method according to claim 7, wherein in step f), said denaturing solution comprises the following components:
i. Guanidinium-HCl, 1-8 M, preferably 3-6 M, more preferably 4 M, Cysteine, 1-100 mM, preferably 5 mM,
ii. Tris, 0.001-1M, preferably 0.1 M,
iii. EDTA, 1-50 mM, preferably 10 mM,
and has a pH between 7 and 10, preferably of 8.

9. The method according to claim 7 or 8, wherein in step g) said refolding solution comprises:
i. a chaperone, preferably Arginine, preferably at a concentration of 0.5-1.0 M, more preferably 0.75 M,
ii. a metal chelator, preferably EDTA, preferably at a concentration of 1-10 mM, more preferably 5 mM,
iii. a redox shuffling system, preferably selected from a combination of L-Cystine and L- Cysteine and a combination of oxidized glutathione and reduced glutathione, more preferably selected from a combination of 1 mM L-Cystine and 5 mM L- Cysteine and a combination of 1 mM GSSG (oxidized glutathione) and 5 mM GSH (reduced glutathione);
and has a pH between 8 and 11.

10. The method according to any one of claims 6 to 9, wherein the solution of purified human proBDNF mutein obtained in step (ii) comprises urea, preferably comprises citric acid and urea.

11. The method according to any one of claims 6 to 10, wherein step (ii) comprises purifying the human proBDNF mutein from the solution provided in step (i) via chromatographic purification.

12. The method according to claim 11, wherein in said chromatographic purification an eluent solution comprising urea, preferably comprising citric acid and urea, is used.

13. The method according to any one of claims 6 to 12, wherein in step (iii) said cleaving comprises adjusting the pH of the solution of the purified human proBDNF mutein obtained in step (ii) to a pH value between 5.8 and 9 and cleaving the human proBDNF mutein in said solution to obtain human BDNF.

14. The method according to any one of claims 6 to 13, wherein in step (iii) said cleaving is carried out by a trypsin-like protease.

15. The method according to claim 14, wherein the ratio of trypsin-like protease to human proBDNF mutein is between 1 : 20000 - 1 : 30000 w/w, preferably between 1 : 23000 - 1 : 27000 w/w, more preferably the ratio is 1 : 25000 w/w.

16. The method according to any one of claims 6 to 15, further comprising step (iv) of purifying the human BDNF obtained in step (iii).
